# EUROPÄISCHE PATENTSCHRIFT

(11) **EP 1 461 034 B1**
(45) Veröffentlichungstag und Bekanntmachung des Hinweises auf die Patenterteilung: **22.11.2006**
(21) Anmeldenummer: 02796586.2
(22) Anmeldetag: 09.12.2002
(51) Int. Cl.: A61K 31/4168, A61K 31/4184, A61P 11/00, A61P 25/00, A61P 33/14, A61P 43/00, C07D 233/50, C07D 235/02, C07D 235/30

(54) **SUBSTITUIERTE IMIDAZOLIDINE, VERFAHREN ZU IHRER HERSTELLUNG, IHRE VERWENDUNG ALS MEDIKAMENT ODER DIAGNOSTIKUM SOWIE ENTHALTENDES MEDIKAMENT**
SUBSTITUTED IMIDAZOLIDINES, METHOD FOR THE PRODUCTION THEREOF, USE THEREOF AS A DRUG OR FOR DIAGNOSIS, AND DRUG CONTAINING SUBSTITUTED IMIDAZOLIDINES
IMIDAZOLIDINES SUBSTITUEES, PROCEDE DE PRODUCTION DE CES IMIDAZOLIDINES, LEUR UTILISATION COMME MEDICAMENT OU EN DIAGNOSTIC ET MEDICAMENT CONTENANT CES IMIDAZOLIDINES

(30) Priorität: 21.12.2001 DE 10163239
(43) Veröffentlichungstag der Anmeldung: 29.09.2004
(73) Patentinhaber: Sanofi-Aventis Deutschland GmbH, 65929 Frankfurt am Main (DE)
(72) Erfinder: HEINELT, Uwe, 65187 Wiesbaden (DE); LANG, Hans-Jochen, 65719 Hofheim (DE); HOFMEISTER, Armin, 55276 Oppenheim (DE); WIRTH, Klaus, 65830 Kriftel (DE)
(86) Internationale Anmeldenummer: PCT/EP2002/013921
(87) Internationale Veröffentlichungsnummer: WO 2003/053434

(56) Entgegenhaltungen:
- WO-A-02/46169
- TURNER J R ET AL: "Transepithelial resistance can be regulated by the intestinal brush-border Na(+)/H(+) exchanger NHE3." AMERICAN JOURNAL OF PHYSIOLOGY. CELL PHYSIOLOGY. UNITED STATES DEC 2000, Bd. 279, Nr. 6, Dezember 2000 (2000-12), Seiten C1918-C1924, XP002233332 ISSN: 0363-6143

## Beschreibung

Substituierte Imidazolidine, Verfahren zu ihrer Herstellung, ihre Verwendung als Medikament oder Diagnostikum sowie sie enthaltendes Medikament

Die Erfindung betrifft substituierte Imidazolidine der Formel I, 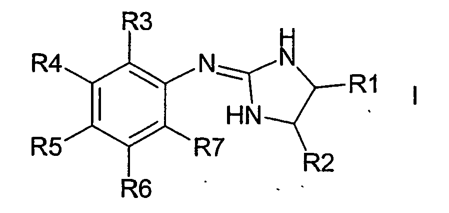 worin bedeuten:
- R1 und R2: unabhängig voneinander CN, (C₁-C₅)-Alkyl, (C₂-C₅)-Alkenyl, (C₂-C₅)-Alkinyl, (C₃-C₆)-Cycloalkyl oder (C₄-C₆)-Cycloalkenyl
wobei alle Kohlenstoffketten und -ringe unsubstituiert sind oder unabhängig voneinander substituiert sind mit 1 - 11 F-Atomen oder mit bis zu zwei Resten ausgewählt aus der Gruppe bestehend aus OH, NH₂, NHCH₃, N(CH₃)₂ und OCH₃;
oder
- R1 und R2: zusammen mit den beiden Kohlenstoffatomen, an die sie gebunden sind, einen fünf- bis achtgliedrigen gesättigten oder ungesättigten Kohlenstoffring,
wobei aber keine Doppelbindung zwischen den beiden Kohlenstoffatomen liegt, an die R1 und R2 gebunden sind und
wobei der Ring unsubstituiert ist oder durch 1 - 12 F-Atome oder bis zu zwei Reste ausgewählt aus der Gruppe bestehend aus CH₃ und OCH₃ substituiert ist;
- R3: F, Cl, Br, I, (C₁ -C₄)-Alkyl, (C₁-C₄)-Alkenyl, (C₃-C₆)-Cycloalkyl, OH, (C₁-C₄)-Alkoxy, OPhenyl, CN, NO₂ oder NH₂;
wobei Phenyl unsubstituiert ist oder substituiert mit bis zu zwei Resten ausgewählt aus der Gruppe bestehend aus CH₃, F, Cl, Br, I, OH und
OCH₃;
und
wobei die Kohlenstoffketten oder -ringe unsubstituiert sind oder substituiert sind mit 1 - 11 F-Atomen;
- R4 bis R6: unabhängig voneinander H, F, Cl, Br, l, (C₁-C₄)-Alkyl, (C₁-C₄)-Alkenyl, (C₃₋C₆)-Cycloalkyl, OH, (C₁-C₄)-Alkoxy, CN, NO₂, NH₂, (C₁-C₄)-Alkylamino oder (C₁-C₄)-Dialkylamino;
wobei die Kohlenstoffketten oder -ringe unsubstituiert sind oder substituiert sind mit 1 - 11 F-Atomen;
- R7: H, F, Cl, Br, I, (C₁-C₄)-Alkyl, (C₁-C₄)-Alkenyl, (C₃-C₆)-Cycloalkyl, OH, (C₁₋C₄)-Alkoxy, CN, NO₂ oder NH₂;
wobei die Kohlenstoffketten oder -ringe unsubstituiert sind oder substituiert sind mit 1 - 11 F-Atomen;
sowie deren pharmazeutisch verträgliche Salzen, sowie die Trifluoressigsäuresalze.

Bevorzugt sind Verbindungen der Formel I, worin bedeuten:
- R1 und R2: unabhängig voneinander (C₁-C₅)-Alkyl, (C₂-C₅)-Alkenyl, (C₂-C₅)-Alkinyl, (C₃₋C₆)-Cycloalkyl oder (C₄-C₆)-Cycloalkenyl,
wobei alle Kohlenstoffketten und -ringe unsubstituiert sind oder unabhängig voneinander substituiert sind mit 1 - 11 F-Atomen oder mit bis zu zwei Resten ausgewählt aus der Gruppe bestehend aus NHCH₃, N(CH₃)₂ und OCH₃;
oder
- R1 und R2: zusammen mit den beiden Kohlenstoffatomen, an die sie gebunden sind, einen fünf- bis achtgliedrigen gesättigten oder ungesättigten Kohlenstoffring,
wobei aber keine Doppelbindung zwischen den beiden Kohlenstoffatomen liegt, an die R1 und R2 gebunden sind und
wobei der Ring unsubstituiert ist oder durch 1 - 12 F-Atome oder bis zu zwei Reste ausgewählt aus der Gruppe bestehend aus CH₃ und OCH₃ substituiert ist;
- R3: F, Cl, Br, I, (C₁ -C₄)-Alkyl, (C₁-C₄)-Alkenyl, (C₃-C₆)-Cycloalkyl, OH, (C₁-C₄)-Alkoxy, OPhenyl, CN, NO₂ oder NH₂;
wobei Phenyl unsubstituiert ist oder substituiert mit bis zu zwei Resten ausgewählt aus der Gruppe bestehend aus CH₃, F, Cl, Br, OH und
OCH₃;
und
wobei die Kohlenstoffketten oder -ringe unsubstituiert sind oder substituiert sind mit 1 - 11 F-Atomen;
- R4 bis R6: unabhängig voneinander H, F, Cl, Br, CH₃, OH, OCH₃, CN, NO₂, NH₂, NHCH₃, N(CH₃)₂;
wobei die Methylgruppen unsubstituiert sind oder substituiert sind mit 1 - 3 F-Atomen;
- R7: H, F, Cl, Br, I, (C₁-C₄)-Alkyl, (C₁-C₄)-Alkenyl, (C₃-C₆)-Cycloalkyl, OH, (C₁₋C₄)-Alkoxy, CN, NO₂ oder NH₂;
wobei die Kohlenstoffketten oder -ringe unsubstituiert sind oder substituiert sind mit 1 - 11 F-Atomen;
sowie deren pharmazeutisch verträgliche Salzen, sowie die Trifluoressigsäuresalze.

Ganz besonders bevorzugt sind folgende Verbindungen der Formel I:
trans-(2-Chlor-6-trifluoromethyl-phenyl)-(octahydro-benzoimidazol-2-yliden)-amin-Hydrochlorid,
(S,S)-(2,6-Dichlorphenyl)-(octahydro-benzoimidazol-2-yliden)-amin-Trifluoressigsäuresalz,
cis-(2,6-Dichlorphenyl)-(octahydro-benzoimidazol-2-yliden)-amin-Trifluoressigsäuresalz,
(R,R)-(2,6-Dichlorphenyl)-(octahydro-benzoimidazol-2-yliden)-amin-Trifluoressigsäuresalz,trans-(Octahydro-benzoimidazol-2-yliden)-(2-phenoxy-phenyl)-amin-Hydrochlorid,
trans-(2,6-Dichlorphenyl)-(4,5-diisopropyl-imidazolidin-2-yliden)-amin-Hydrochlorid,
trans-(2,6-Dichlorphenyl)-(4,5-dicyclopropyl-imidazolidin-2-yliden)-amin-Trifluoressigsäuresalz,cis-(2,6-Dichlorphenyl)-(4,5-dicyclopropyl-imidazolidin-2-yliden)-amin-Hydrochlorid,
trans-(2,6-Dichloro-phenyl)-(4,5-diethyl-imidazolidin-2-yliden)-amin-Hydrochlorid,
(2,6-Dichloro-phenyl)-(4,5-dimethyl-imidazolidin-2-yliden)-amin-Salpetersäuresalz,
trans-(2,6-Dichloro-phenyl)-(hexahydro-cyclopentaimidazol-2-yliden)-amin-Trifluoressigsäuresalz.

Ganz außerordentlich besonders bevorzugt sind folgende Verbindungen der Formel I:
(S,S)-(2,6-Dichlorphenyl)-(octahydro-benzoimidazol-2-yliden)-amin-Trifluoressigsäuresalz,
cis-(2,6-Dichlorphenyl)-(octahydro-benzoimidazol-2-yliden)-amin-Trifluoressigsäuresalz,
(R,R)-(2,6-Dichlorphenyl)-(octahydro-benzoimidazol-2-yliden)-amin-Trifluoressigsäuresalz,
trans-(2,6-Dichlorphenyl)-(4,5-diisopropyl-imidazolidin-2-yliden)-amin-Hydrochlorid,trans-(2,6-Dichlorphenyl)-(4,5-dicyclopropyl-imidazolidin-2-yliden)-amin-Trifluoressigsäuresalz,cis-(2,6-Dichlorphenyl)-(4,5-dicyclopropyl-imidazolidin-2-yliden)-amin-Hydrochlorid,trans-(2,6-Dichloro-phenyl)-(4,5-diethyl-imidazolidin-2-yliden)-amin-Hydrochlorid,(2,6-Dichloro-phenyl)-(4,5-dimethyl-imidazolidin-2-yliden)-amin-Salpetersäuresalz,
trans-(2,6-Dichloro-phenyl)-(hexahydro-cyclopentaimidazol-2-yliden)-amin-Trifluoressigsäuresalz.

Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate. Diese Gruppe entspricht auch den physiologisch akzeptablen Anionen; aber auch Trifluoracetate.

Enthalten die Verbindungen der Formel 1 ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Verbindungen der Formel I können weiterhin als Tautomere oder als Gemisch tautomerer Strukturen vorliegen.
Dabei ist vor allem an folgende Tautomere gedacht: 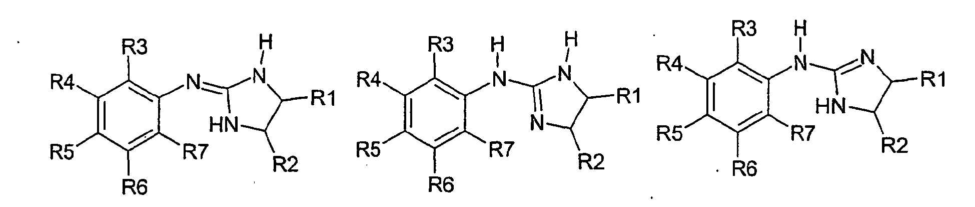 Sind R1 und R2 verschieden und verfügt die Stickstoff-Kohlenstoff-Doppelbindung über ausreichende konfigurative Stabilität, so kommen auch noch zwei Doppelbindungsisomere in Betracht:

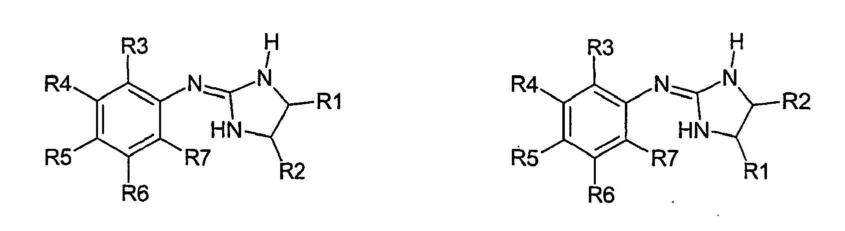

Die bezeichneten Kohlenstoffreste bzw. teilweise oder vollständig fluorierten oder substituierten Kohlenstoffreste können sowohl geradkettig als auch verzweigt vorliegen.

Beschrieben werden auch Methoden zur Herstellung der verwendeten Verbindungen. So lassen sich die durch Formel I beschriebenen Substanzen in dem Fachmann bekannter Weise aus den zugrundeliegenden Isothiocyanaten II und den entsprechenden Diaminen III herstellen.

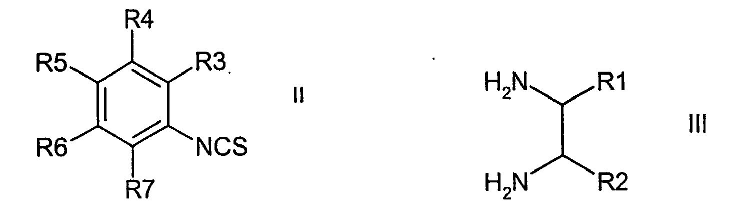

Das hierbei intermediär gebildete Thioharnstoff-Derivat wird mittels Methyliodid (Synthesis, 1974, 41-42) oder Carbodiimid (Synthesis, 1977, 864 - 865) zum entsprechenden Imidazolidin I cyclisiert. Die hierbei verwendeten Isothiocyanate II können, falls nicht käuflich erhältlich, in literaturbekannter Weise aus den entsprechenden Anilinen durch dem Fachmann bekannte Methoden, z. B. durch Behandeln mit Thiophosgen (J. Med. Chem., 1975, 18, 90-99) oder Thiocarbonyldiimidazol (Justus Liebigs Ann. Chem., 1962, 657, 104) hergestellt werden.

Neben den oben beschriebenen Isothiocyanaten II lassen sich auch die Isocyanate IV erfolgreich mit Aminen vom Typ der Formel III zu Verbindungen der Formel I umsetzen. Dabei wird das intermediär gebildete Harnstoffderivat mit Phosphoroxychlorid zu den entsprechenden Imidazolidinen der Formel I cyclisiert.

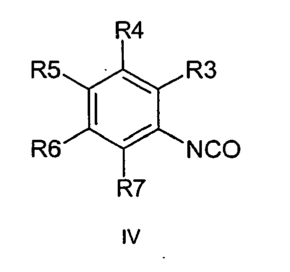

Bei der vorliegenden Erfindung konnte überraschend gezeigt werden, dass die beschriebenen Verbindungen potente Inhibitoren des Natrium-/ Wasserstoff-Austauschers (NHE) darstellen, insbesondere des NHE3.

Die bisherigen uns bekannten NHE3-Inhibitoren leiten sich von Verbindungen des Acylguanidin-Typs (EP-OS 825 178, HOE 96/F226), Norbornylamin-Typs (DE 199 60 204.2-HMR 99 /L 073), 2-Guanidino-chinazolin-Typs (WO 01 79 186 A1 oder Benzamidin-Typs (WO 01 21582 A1, WO 01 72 742 A1) ab. Das ebenfalls als NHE3-Inhibitor beschriebene Squalamin (M. Donowitz et al. Am. J. Physiol. 276 (Cell Physiol. 45): C136 - C144) wirkt nicht unmittelbar wie die Verbindungen nach Formel I, sondern erreicht seine maximale Wirkstärke erst nach einer Stunde. WO 02/46169 offenbart substituierte 2-Anilino-Benzimidazole und ihre Verwendung als NHE-Inhibitoren.

Das den hier beschriebenen Verbindungen ähnliche Clonidin ist als schwacher NHE-Inhibitor bekannt. Allerdings ist seine Wirkung auf den NHE3 der Ratte mit einer IC₅₀ von 620 µM äußerst moderat. Stattdessen weist es eine gewisse Selektivität für den NHE2 auf, an dem es eine IC₅₀ von 42 µM besitzt (J. Orlowski et al J. Biol. Chem. 268, 25536). Es ist daher eher als NHE2-Inhibitor zu bezeichnen. Neben der schwachen NHE-Wirkung besitzt das Clonidin eine hohe Affinität zum adrenergen alpha2-Rezeptor und imidazolin I1-Rezeptor, wodurch eine starke blutdrucksenkende Wirkung vermittelt wird (Ernsberger et al Eur. J. Pharmacol. 134, 1, 1987). Verbindungen der Formel I zeichnen sich durch gesteigerte NHE3-Aktivität und reduzierte I1- und alpha2-Aktivität aus.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund dieser unerwarteten Eigenschaft eignen sich die Verbindungen der Formel I zur Behandlung von Krankheiten, die durch Sauerstoffmangel hervorgerufen werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.

Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende, und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg/kg pro Tag notwendig werden.

### Versuchsbeschreibungen und Beispiele:

Liste der verwendeten Abkürzungen:
- Rt: Retentionszeit
- TFA: Trifluoressigsäure
- LCMS: Liquid Chromatography Mass Spectroscopy
- MS: Mass Spectroscopy
- Cl⁺: Chemical lonization, positive mode
- ES⁺: Electrospray, positive mode

### Allgemeines:

Die im Folgenden angegebenen Retentionszeiten (Rt) beziehen sich auf LCMS-Messungen mit den folgenden Parametern:

### Methode A:

| | |
|---|---|
| stationäre Phase: | Merck Purospher 3µ 2 x 55 mm |
| mobile Phase: | 95% H₂O (0,05% TFA)→ 95% Acetonitril; 4 min; 95% |
| | Acetonitril; 1,5 min → 5% Acetonitril; 1 min; 0,5 ml/min. |
| Methode B: | |
| stationäre Phase: | YMC J'sphereODS H80 2 x 33 mm |
| mobile Phase: | 95% H₂O (0,05% TFA)→ 95% Acetonitril; 2,3 min; 95% |
| | Acetonitril; 1 min → 5% Acetonitril; 0,1 min; 1 ml/min. |

Die präparative HPLC wurde unter folgenden Bedingungen durchgeführt:

| | |
|---|---|
| stationäre Phase: | Merck Purospher RP18 (10µM) 250 x 25 mm |
| mobile Phase: | 90% H₂O (0,05% TFA)→ 90% Acetonitril; 40 min; 25 ml/min |

Handelt es sich um enantiomerenreine Verbindungen, so ist die Konfiguration und/oder das Vorzeichen der optischen Drehung angegeben. Fehlen diese Angaben, so handelt es sich um Racemate oder optisch nicht aktive Verbindungen.

### Beispiel 1: (S,S)-(2,6-Dichlorphenyl)-(octahydro-benzoimidazol-2-yliden)-amin-Trifluoressigsäuresalz

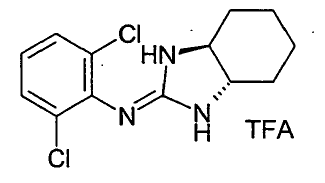

2,6-Dichlorphenyl-isothiocyanat (600 mg) und (1S,2S)-(+)-1,2-Diaminocyclohexan (336 mg) wurden in Toluol (30 ml) gelöst und 3h bei 70 °C gerührt. Nach Stehenlassen über Nacht wurde das Lösungsmittel abgezogen und der Rückstand mit Ether versetzt. Anschließend wurde der entstandene Thioharnstoff abgesaugt. Es wurden 840 mg des gewünschten Produkts isoliert.
Ein Teil des so gewonnenen Thioharnstoffs (420 mg) wurde dann mit Toluol (15 ml) versetzt und kurz auf Rückfluss erhitzt. Anschließend wurde N,N'-Dicyclohexylcarbodiimid (226 mg) gelöst in Toluol (5 ml) zugetropft und das Gemisch 5 h bei 70°C gerührt. Nach Stehenlassen über Nacht wurde der gebildete Niederschlag abfiltriert und das Filtrat zur Trockne eingeengt. Der Rückstand wurde dann über präparative HPLC gereinigt. Die sauberen Fraktionen wurden vereinigt, das Acetonitril am Rotationsverdampfer abgezogen und die wässerige Phase gefriergetrocknet. Es wurden 70 mg der gewünschten Verbindung erhalten.
LCMS-Rt: 3,69 min, (A)
MS (ES⁺, M+H⁺): 284,2

### Beispiel 2: cis-(2,6-Dichlorphenyl)-(octahydro-benzoimidazol-2-yliden)-amin-Trifluoressigsäuresalz

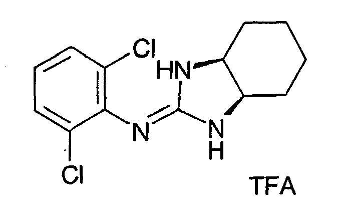

2,6-Dichlorphenyl-isothiocyanat (600 mg) und cis-1,2-Diaminocyclohexan (336 mg) wurden, wie in Beispiel 1 beschrieben, umgesetzt und aufgearbeitet. Von den im ersten Schritt erhaltenen 900 mg Thioharnstoff wurden im nächsten 454 mg weiter umgesetzt. Es wurden 112 mg der gewünschten Verbindung erhalten.
LCMS-Rt: 3,65 min, (A)
MS (Cl⁺, M+H⁺): 284,1

### Beispiel 3: (R,R)-(2,6-Dichlorphenyl)-(octahydro-benzoimidazol-2-yliden)-amin-Trifluoressigsäuresalz

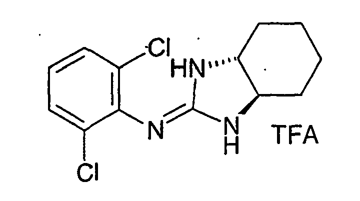

2,6-Dichlorphenyl-isothiocyanat (50 mg) und (R,R)-(-)-1,2-Diaminocyclohexan (28 mg) wurden in Toluol (1,5 ml) vorgelegt und 15 min auf Rückfluss erhitzt. Anschließend wurde N,N'-Dicyclohexylcarbodiimid (76 mg) zugesetzt und weiter am Rückfluss gehalten. Nach Stehenlassen über Nacht wurde das Toluol abgezogen und der Rückstand mittels präparativer HPLC gereinigt. Da bei der ersten Reinigung nur verunreinigte Fraktionen erhalten wurden, wurde die Chromatographie mit einer anderen Säule (MN Nucleosil 100-5-C18 250 x 25 mm; Fluss 20 ml/min), aber ansonsten gleichen Bedingungen wiederholt. Die sauberen Fraktionen wurden vereinigt, das Acetonitril am Rotationsverdampfer abgezogen und die wässerige Phase gefriergetrocknet. Es wurden 10 mg der gewünschten Verbindung erhalten.
LCMS-Rt: 3,70 min, (A)
MS (Cl⁺, M+H⁺): 284,0

### Beispiel 4: trans-(Octahydro-benzoimidazol-2-yliden)-(2-phenoxy-phenyl)-amin-Hydrochlorid

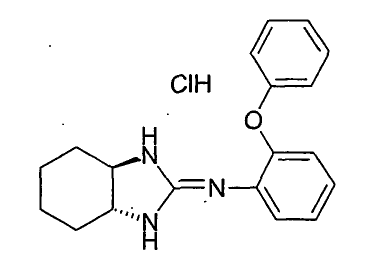

### a) 2-Phenoxyphenylisothiocyanat

Zu einer Lösung aus 1,85 g (0,01 mol) 2-Phenoxyanilin in 50 ml THF gab man 1,96 g (0,011 mol) Thiocarbonyldiimidazol, rührte 4 Stunden bei Raumtemperatur und erhielt die Verbindung nach Abdestillieren des Lösungsmittels als braunes amorphes Produkt.

### b) N-(trans-2-Aminocyclohexyl)-N'-(2-phenoxyphenyl)-thioharnstoff

Eine Lösung von 0,8 g trans-1,2-Diaminocyclohexan in 30 ml THF wurde mit einer Lösung aus 1,6 g 2-Phenoxyphenylisothiocyanat in 10 ml THF versetzt und etwa 4 Stunden bei Raumtemperatur gerührt. Nach Verdampfen des Lösungsmittels und nachfolgender Säulenchromatografie an Kieselgel mit einem Gemisch aus 10 Teilen Ethylacetat, 5 Teilen n-Heptan, 5 Teilen Methylenchlorid, 5 Teilen Methanol und 1 Teil konzentrierter wässriger Ammoniak-Lösung erhielt man die gewünschter Verbindung als amorphes, öliges Produkt.

### c) trans-(Octahydro-benzoimidazol-2-yliden)-(2-phenoxy-phenyl)-amin-Hydrochlorid

Eine Lösung von 1,03 g N-(trans-2-Aminocyclohexyl)-N'-(2-phenoxyphenyl)-thioharnstoff in 30 ml Ethanol versetzte man mit 3,4 g Methyliodid und hielt die Reaktionsmischung 5 Stunden am Rückfluss. Nach dem Stehenlassen über Nacht destillierte man das Lösungsmittel ab und behandelte den Rückstand mit Wasser und stellte anschließend mit gesättigter Natriumbicarbonatlösung alkalisch. Nach Extraktion der wässrigen Phase mit Ethylacetat und Verdampfen der organischen Extraktionsphase wurde der ölige Rückstand an Kieselgel mit einem Gemisch aus 10 Teilen Ethylacetat, 5 Teilen n-Heptan, 5 Teilen Methylenchlorid, 5 Teilen Methanol und 1 Teil konzentrierter wässeriger Ammoniak-Lösung chromatografiert. Man erhielt ein öliges Produkt, das in Essigester gelöst und mit einer gesättigten Lösung von HCl-Gas in Diethylether sauer gestellt wurde. Nach Abdestillieren des Lösungsmittels wurde in Wasser gelöst und einer Gefriertrocknung unterworfen. Man erhielt 0,49 g eines Feststoffs vom Schmp. 110°C.
MS (ES⁺, M+H⁺): 308,2

### Beispiel 5: trans-(2-Chlor-6-trifluoromethyl-phenyl)-(octahydro-benzoimidazol-2-yliden)-amin-Hydrochlorid

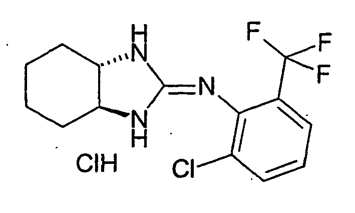

### a) N-(trans-2-Aminocyclohexyl)-N'-(2-chlor-6-trifluormethylphenyl)-harnstoff

Eine Lösung von 1,6 g 2-Chlor-6-trifluormethylphenylisocyanat in 30 ml THF wurde mit einer Lösung aus 0,46 g trans-1,2-Diaminocyclohexan in 10 ml THF versetzt und etwa 3 Stunden bei Raumtemperatur gerührt. Nach dem Stehenlassen über Nacht wurde das Lösungsmittel abdestilliert und 0,57 g der gewünschten Verbindung als halbfestes, gelbes Produkt erhalten.

### b) trans-(2-Chlor-6-trifluoromethyl-phenyl)-(octahydro-benzoimidazol-2-yliden)-amin-Hydrochlorid

0,57 g N-(trans-2-Aminocyclohexyl)-N'-(2-chlor-6-trifluormethylphenyl)-harnstoff wurden in 20 ml Phosphoroxidchlorid (POCl₃) für 4-5 Stunden am Rückflußkühler gekocht. Man destillierte das POCl₃ ab, versetzte den Rückstand mit Wasser und stellte mit 2N NaOH auf pH 7 - 8. Man extrahierte sodann mit Ethylacetat, destillierte das organische Lösungsmittel ab und chromatografierte den Rückstand an Kieselgel mit einem Gemisch aus 20 Teilen Ethylacetat, 10 Teilen n-Heptan und 3 Teilen Eisessig. Nach dem Abdestillieren des Elutionsmittels wurde der weiße feste Rückstand in wenig Ethylacetat gelöst und mit einer gesättigten Lösung von HCl-Gas in Diethylether sauer gestellt. Nach Abdestillieren des Lösungsmittels und Behandeln des Rückstands mit Diisopropylether erhielt man 0,4 g gewünschtes Produkt als Feststoff vom Schmp. 160 -165 °C.
MS (Cl⁺, M+H⁺): 318,3

### Beispiel 6: trans-(4,5-Di-tert-butyl-imidazolidin-2-ylidene)-(2,6-dichlorphenyl)-amin-Hydrochlorid

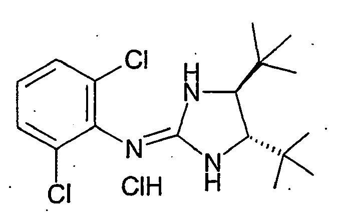

2,6-Dichlorphenyl-isothiocyanat (150 mg) und trans-2,2,5,5-Tetramethyl-hexan-3,4-diamin (127 mg) - analog Synthesis 1999, 2, 228; in racemischer Form - wurden in Toluol (1,5 ml) vorgelegt und 15 min zum Rückfluss erhitzt. Anschließend wurde N,N'-Dicyclohexylcarbodiimid (126 mg), gelöst in 2 ml Toluol, zugesetzt und weiter am Rückfluss gehalten. Nach Stehenlassen über Nacht wurde das Toluol abgezogen und der Rückstand mittels präparativer HPLC gereinigt. Die sauberen Fraktionen wurden vereinigt, das Acetonitril am Rotationsverdampfer abgezogen, die wässrige Phase mit gesättigter Kaliumcarbonat-Lösung neutralisiert und dreimal mit Essigester extrahiert. Die vereinigten Essigester-Phasen wurden mit gesättigter Natriumchlorid-Lösung gewaschen und dann über Magnesiumsulfat getrocknet. Nach Abfiltrieren des Trockenmittels und Einengen wurde der Rückstand mit Wasser aufgenommen, 2 normale Salzsäure zugegeben und gefriergetrocknet. Es wurden 111 mg der gewünschten Verbindung erhalten.
LCMS-Rt: 4,43 min, (A)
MS (Cl⁺, M+H⁺): 342,2

### Beispiel 7: trans-(2,6-Dichlorphenyl)-(4,5-diisopropyl-imidazolidin-2-yliden)-amin-Hydrochlorid

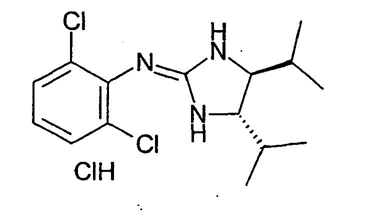

Trans-2,5-Dimethyl-hexan-3,4-diamin (226 mg) - analog Synthesis 1999, 2, 228; in racemischer Form -wurde in THF (2,5 ml) vorgelegt und 2,6-Dichlorphenylisothiocyanat portionsweise bei Raumtemperatur zuggegeben (150, 80 und 40 mg-Portionen). Anschließend wurde N,N'-Dicyclohexylcarbodiimid (324 mg) zugesetzt und weiter bei Raumtemperatur gerührt. Zur Vervollständigung der Reaktion wurde noch etwas N,N'-Dicyclohexylcarbodiimid zugegeben. Nach Stehenlassen über Nacht wurde der entstandene Niederschlag abgesaugt und das Filtrat eingeengt. Der Rückstand wurde mittels präparativer HPLC gereinigt. Die sauberen Fraktionen wurden vereinigt, das Acetonitril am Rotationsverdampfer abgezogen, die wässrige Phase mit gesättigter Kaliumcarbonat-Lösung neutralisiert und dreimal mit Essigester extrahiert. Die vereinigten Essigester-Phasen wurden mit gesättigter Natriumchlorid-Lösung gewaschen und dann über Magnesiumsulfat getrocknet. Nach Abfiltrieren des Trockenmittels und Einengen wurde der Rückstand mit Wasser aufgenommen, 2 normale Salzsäure zugegeben und gefriergetrocknet..Es wurden 220 mg der gewünschten Verbindung erhalten.
LCMS-Rt: 1,93 min, (B)
MS (ES⁺, M+H⁺): 314,1

Die in nachfolgender Tabelle beschriebenen Verbindungen wurden entsprechend dem jeweils angegebenen Beispiel synthetisiert:

| Beispiel | | Salz | Analog Beispiel | MS [M+H⁺] | LCMS-Rt [min] |
|---|---|---|---|---|---|
| 8 | 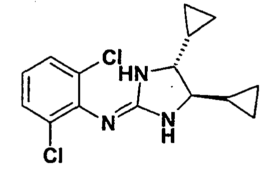 | TFA | 7 | 309,0 (ES⁺) | 1,84 (B) |
| 9 | 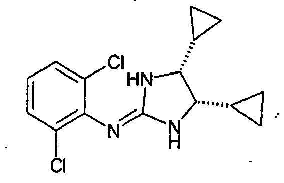 | HCl | 7 | 309,1 (ES⁺) | 1,87 (B) |
| 10 | 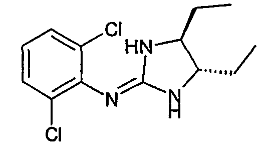 | HCl | 7 | 286,1 (ES⁺) | 1,75 (B) |
| 11 | 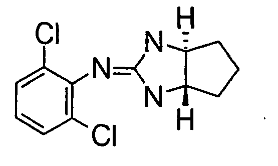 | TFA | 7 | 270,1 (ES⁺) | 1,56 (B) |
| 12 | 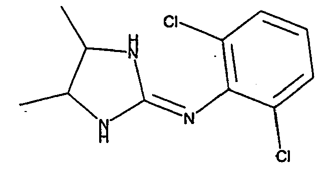 | HNO₃ | | | |

### Pharmakologische Daten:

### Testbeschreibung:

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M KOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC-Wert der jeweiligen Substanz für die einzelnen NHE-Subtypen berechnet.

**Ergebnisse:**

| Beispiel | IC₅₀ [µM], (rNHE3) |
|---|---|
| 5 | 19 |
| 7 | 1,1 |
| 12 | -3 |

## Patentansprüche

1. Imidazolidine der Formel 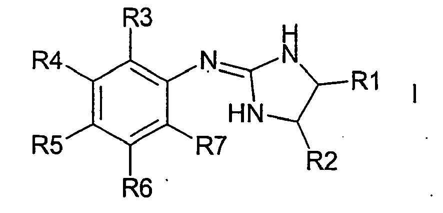 worin bedeuten:
R1 und R2 unabhängig voneinander CN, (C₁-C₅)-Alkyl, (C₂-C₅)-Alkenyl, (C₂-C₅)-Alkinyl, (C₃-C₆)-Cycloalkyl oder (C₄-C₆)-Cycloalkenyl
wobei alle Kohlenstoffketten und -ringe unsubstituiert sind oder unabhängig voneinander substituiert sind mit 1 - 11 F-Atomen oder mit bis zu zwei Resten ausgewählt aus der Gruppe bestehend aus OH, NH₂, NHCH₃, N(CH₃)₂ und OCH₃;
oder
R1 und R2 zusammen mit den beiden Kohlenstoffatomen, an die sie gebunden sind, einen fünf- bis achtgliedrigen gesättigten oder ungesättigten Kohlenstoffring,
wobei aber keine Doppelbindung zwischen den beiden Kohlenstoffatomen liegt, an die R1 und R2 gebunden sind und
wobei der Ring unsubstituiert ist oder durch 1 - 12 F-Atome oder bis zu zwei Reste ausgewählt aus der Gruppe bestehend aus CH₃ und OCH₃ substituiert ist;
R3 F, Cl, Br, I, (C₁ -C₄)-Alkyl, (C₁-C₄)-Alkenyl, (C₃-C₆)-Cycloalkyl, OH, (C₁-C₄)-Alkoxy, OPhenyl, CN, NO₂ oder NH₂;
wobei Phenyl unsubstituiert ist oder substituiert mit bis zu zwei Resten ausgewählt aus der Gruppe bestehend aus CH₃, F, Cl, Br, I, OH und OCH₃;
und
wobei die Kohlenstoffketten oder -ringe unsubstituiert sind oder substituiert sind mit 1 - 11 F-Atomen;
R4 bis R6 unabhängig voneinander H, F, Cl, Br, I, (C₁-C₄)-Alkyl, (C₁-C₄)-Alkenyl, (C₃₋C₆)-Cycloalkyl, OH, (C₁-C₄)-Alkoxy, CN, NO₂, NH₂, (C₁-C₄)-Alkylamino oder (C₁-C₄)-Dialkylamino;
wobei die Kohlenstoffketten oder -ringe unsubstituiert sind oder substituiert sind mit 1 - 11 F-Atomen;
R7 H, F, Cl, Br, I, (C₁-C₄)-Alkyl, (C₁-C₄)-Alkenyl, (C₃-C₆)-Cycloalkyl, OH, (C₁₋C₄)-Alkoxy, CN, NO₂ oder NH₂;
wobei die Kohlenstoffketten oder -ringe unsubstituiert sind oder substituiert sind mit 1 - 11 F-Atomen;
sowie deren pharmazeutisch verträgliche Salzen, sowie die Trifluoressigsäuresalze.

2. Verbindungen der Formel I nach Anspruch 1, worin bedeuten:
R1 und R2 unabhängig voneinander (C₁-C₅)-Alkyl, (C₂-C₅)-Alkenyl, (C₂-C₅)-Alkinyl, (C₃₋C₆)-Cycloalkyl oder (C₄-C₆)-Cycloalkenyl,
wobei alle Kohlenstoffketten und -ringe unsubstituiert sind oder unabhängig voneinander substituiert sind mit 1 - 11 F-Atomen oder mit bis zu zwei Resten ausgewählt aus der Gruppe bestehend aus NHCH₃, N(CH₃)₂ und OCH₃;
oder
R1 und R2 zusammen mit den beiden Kohlenstoffatomen, an die sie gebunden sind, einen fünf- bis achtgliedrigen gesättigten oder ungesättigten Kohlenstoffring,
wobei aber keine Doppelbindung zwischen den beiden Kohlenstoffatomen liegt, an die R1 und R2 gebunden sind und wobei der Ring unsubstituiert ist oder durch 1 - 12 F-Atome oder bis zu zwei Reste ausgewählt aus der Gruppe bestehend aus CH₃ und OCH₃ substituiert ist;
R3 F, Cl, Br, I, (C₁ -C₄)-Alkyl, (C₁-C₄)-Alkenyl, (C₃-C₆)-Cycloalkyl, OH, (C₁-C₄)-Alkoxy, OPhenyl, CN, NO₂ oder NH₂;
wobei Phenyl unsubstituiert ist oder substituiert mit bis zu zwei Resten ausgewählt aus der Gruppe bestehend aus CH₃, F, Cl, Br, OH und OCH₃;
und
wobei die Kohlenstoffketten oder -ringe unsubstituiert sind oder substituiert sind mit 1 - 11 F-Atomen;
R4 bis R6 unabhängig voneinander H, F, Cl, Br, CH₃, OH, OCH₃, CN, NO₂, NH₂, NHCH₃, N(CH₃)₂;
wobei die Methylgruppen unsubstituiert sind oder substituiert sind mit 1 - 3 F-Atomen;
R7 H, F, Cl, Br, I, (C₁-C₄)-Alkyl, (C₁-C₄)-Alkeny (C₃-C₆)-Cycloalkyl, OH, (C₁₋C₄)-Alkoxy, CN, NO₂ oder NH₂;
wobei die Kohlenstoffketten oder -ringe unsubstituiert sind oder substituiert sind mit 1 - 11 F-Atomen;
sowie deren pharmazeutisch verträgliche Salzen, sowie die Trifluoressigsäuresalze.

3. Verbindungen der Formel I nach einem oder mehreren Ansprüchen 1 und 2, welche sind:
trans-(2-Chlor-6-trifluoromethyl-phenyl)-(octahydro-benzoimidazol-2-yliden)-amin-Hydrochlorid,
(S,S)-(2,6-Dichlorphenyl)-(octahydro-benzoimidazol-2-yliden)-amin-Trifluoressigsäuresalz,
cis-(2,6-Dichlorphenyl)-(octahydro-benzoimidazol-2-yliden)-amin-Trifluoressigsäuresalz,
(R,R)-(2,6-Dichlorphenyl)-(octahydro-benzoimidazol-2-yliden)-amin-Trifluoressigsäuresalz,
trans-(Octahydro-benzoimidazol-2-yliden)-(2-phenoxy-phenyl)-amin-Hydrochlorid,
trans-(2,6-Dichlorphenyl)-(4,5-diisopropyl-imidazolidin-2-yliden)-amin-Hydrochlorid,
trans-(2,6-Dichlorphenyl)-(4,5-dicyclopropyl-imidazolidin-2-yliden)-amin-Trifluoressigsäuresalz,
cis-(2,6-Dichlorphenyl)-(4,5-dicyclopropyl-imidazolidin-2-yliden)-amin-Hydrochlorid,
trans-(2,6-Dichloro-phenyl)-(4,5-diethyl-imidazolidin-2-yliden)-amin-Hydrochlorid,
(2,6-Dichloro-phenyl)-(4,5-dimethyl-imidazolidin-2-yliden)-amin-Salpetersäuresalz,
trans-(2,6-Dichloro-phenyl)-(hexahydro-cyclopentaimidazol-2-yliden)-amin-Trifluoressigsäuresalz,

4. Verbindungen der Formel I nach einem oder mehreren Ansprüchen 1 bis 3, welche sind:
(S,S)-(2,6-Dichlorphenyl)-(octahydro-benzoimidazol-2-yliden)-amin-Trifluoressigsäuresalz,
cis-(2,6-Dichlorphenyl)-(octahydro-benzoimidazol-2-yliden)-amin-Trifluoressigsäuresalz,
(R,R)-(2,6-Dichlorphenyl)-(octahydro-benzoimidazol-2-yliden)-amin-Trifluoressigsäuresalz,
trans-(2,6-Dichlorphenyl)-(4,5-diisopropyl-imidazolidin-2-yliden)-amin-Hydrochlorid,
trans-(2,6-Dichlorphenyl)-(4,5-dicyclopropyl-imidazolidin-2-yliden)-amin-Trifluoressigsäuresalz,
cis-(2,6-Diclilorphenyl)-(4,5-dicyclopropyl-imidazolidin-2-yliden)-amin-Hydrochlorid,
trans-(2,6-Dichloro-phenyl)-(4,5-diethyl-imidazolidin-2-yliden)-amin-Hydrochlorid,
(2,6-Dichloro-phenyl)-(4,5-dimethyl-imidazolidin-2-yliden)-amin-Salpetersäuresalz,
trans-(2,6-Dichloro-phenyl)-(hexahydro-cyclopentaimidazol-2-yliden)-amin-Trifluoressigsäuresalz.

5. Verwendung einer Verbindung der Formel I nach Anspruch 1 zur Herstellung eines Medikaments zur Behandlung oder Prophylaxe von Störungen des Atemantriebs.

6. Verwendung einer Verbindung der Formel I nach Anspruch 1 zur Herstellung eines Medikaments zur Behandlung oder Prophylaxe von Atemstörungen, insbesondere Schlaf-bedingten Atemstörungen wie Schlafapnoen.

7. Verwendung einer Verbindung der Formel I nach Anspruch 1 zur Herstellung eines Medikaments zur Behandlung oder Prophylaxe des Schnarchens.

8. Verwendung einer Verbindung der Formel I nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

9. Verwendung einer Verbindung der Formel I nach Anspruch 1 zur Herstellung eines Medikaments zur Behandlung oder Prophylaxe von Störungen der Darmfunktion.

10. Verwendung einer Verbindung der Formel I nach Anspruch 1 zur Herstellung eines Medikaments zur Behandlung oder Prophylaxe von Störungen der Gallenfunktion.

11. Verwendung einer Verbindung der Formel I nach Anspruch 1 zur Herstellung eines . Medikaments zur Behandlung oder Prophylaxe von ischämischen Zuständen des peripheren und zentralen Nervensystems und des Schlaganfalls.

12. Verwendung einer Verbindung der Formel I nach Anspruch 1 zur Herstellung eines Medikaments zur Behandlung oder Prophylaxe von ischämischen Zuständen peripherer Organe und Gliedmaßen.

13. Verwendung einer Verbindung der Formel I nach Anspruch 1 zur Herstellung eines Medikaments zur Behandlung von Schockzuständen.

14. Verwendung einer Verbindung der Formel I nach Anspruch 1 zur Herstellung eines Medikaments zu Einsatz bei chirurgischen Operationen und Organtransplantationen.

15. Verwendung einer Verbindung der Formel I nach Anspruch 1 zur Herstellung eines Medikaments zur Konservierung und Lagerung von Transplantaten für chirurgische Maßnahmen.

16. Verwendung einer Verbindung der Formel I nach Anspruch 1 zur Herstellung eines Medikaments zur Behandlung von Krankheiten, bei denen die Zellproliferation eine primäre oder sekundäre Ursache darstellt.

17. Verwendung einer Verbindung der Formel I nach Anspruch 1 zur Herstellung eines Medikaments zur Behandlung oder Prophylaxe von Störungen des Fettstoffwechsels.

18. Verwendung einer Verbindung der Formel I nach Anspruch 1 zur Herstellung eines Medikaments zur Behandlung oder Prophylaxe des Befalls durch Ektoparasiten.

19. Heilmittel, enthaltend eine wirksame Menge einer Verbindung der Formel I nach einem oder mehreren der Ansprüche 1 bis 3.

## Claims

1. An imidazolidine of the formula 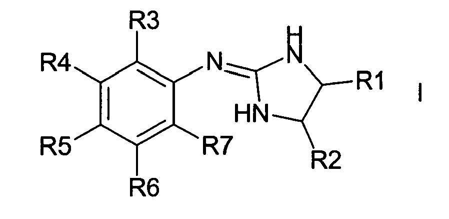 in which
R1 and R2 independently of one another are CN, (C₁-C₅)-alkyl, (C₂-C₅)-alkenyl, (C₂-C₅)-alkynyl, (C₃-C₆)-cycloalkyl or (C₄-C₆)-cycloalkenyl
where all carbon chains and carbon rings are unsubstituted or, independently of one another, are substituted by 1 - 11 fluorine atoms or by up to two radicals selected from the group consisting of OH, NH₂, NHCH₃, N(CH₃)₂ and OCH₃;
or
R1 and R2 together with the two carbon atoms to which they are attached are a five- to eight-membered saturated or unsaturated carbon ring,
but without any double bond between the two carbon atoms to which R1 and R2 are attached, and where the ring is unsubstituted or substituted by 1 - 12 fluorine atoms or by up to two radicals selected from the group consisting of CH₃ and OCH₃;
R3 is F, Cl, Br, I, (C₁-C₄)-alkyl, (C₁-C₄)-alkenyl, (C₃-C₆)-cycloalkyl, OH, (C₁-C₄)-alkoxy, Ophenyl, CN, NO₂ or NH_{2;}
where phenyl is unsubstituted or substituted by up to two radicals selected from the group consisting of CH₃, F, Cl, Br, I, OH and OCH₃;
and
where the carbon chains or carbon rings are unsubstituted or substituted by 1 - 11 fluorine atoms;
R4 to R6 independently of one another are H, F, Cl, Br, I, (C₁-C₄)-alkyl, (C₁-C₄)-alkenyl, (C₃-C₆)-cycloalkyl, OH, (C₁-C₄)-alkoxy, CN, NO₂, NH₂, (C₁-C₄)-alkylamino or (C₁-C₄)-dialkylamino;
where the carbon chains or carbon rings are unsubstituted or substituted by 1 - 11 fluorine atoms;
R7 is H, F, Cl, Br, I, (C₁-C₄)-alkyl, (C₁-C₄)-alkenyl, (C₃-C₆)-cycloalkyl, OH, (C₁-C₄)-alkoxy, CN, NO₂ or NH₂;
where the carbon chains or carbon rings are unsubstituted or substituted by 1 - 11 fluorine atoms;
and its pharmaceutically acceptable salts, and the trifluoroacetic acid salts.

2. A compound of the formula I as claimed in claim 1 in which
R1 and R2 independently of one another are (C₁-C₅)-alkyl, (C₂-C₅)-alkenyl, (C₂-C₅)-alkynyl, (C₃-C₆)-cycloalkyl or (C₄-C₆)-cycloalkenyl,
where all carbon chains and carbon rings are unsubstituted or, independently of one another, substituted by 1 - 11 fluorine atoms or by up to two radicals selected from the group consisting of NHCH₃, N(CH₃)₂ and OCH₃;
or
R1 and R2 together with the two carbon atoms to which they are attached are a five- to eight-membered saturated or unsaturated carbon ring,
but without any double bond between the two carbon atoms to which R1 and R2 are attached,
and
where the ring is unsubstituted or substituted by 1 - 12 fluorine atoms or by up to two radicals selected from the group consisting of CH₃ and OCH₃;
R3 is F, Cl, Br, I, (C₁ -C₄)-alkyl, (C₁-C₄)-alkenyl, (C₃-C₆)-cycloalkyl, OH, (C₁-C₄)-alkoxy, Ophenyl, CN, NO₂ or NH_{2;}
where phenyl is unsubstituted or substituted by up to two radicals selected from the group consisting of CH₃, F, Cl, Br, OH and OCH₃;
and
where the carbon chains or carbon rings are unsubstituted or substituted by 1 - 11 fluorine atoms;
R4 to R6 independently of one another are H, F, Cl, Br, CH₃, OH, OCH₃, CN, NO₂, NH₂, NHCH₃, N(CH₃)₂;
where the methyl groups are unsubstituted or substituted by 1 - 3 fluorine atoms;
R7 is H, F, Cl, Br, I, (C₁-C₄)-alkyl, (C₁-C₄)-alkenyl, (C₃-C₆)-cycloalkyl, OH, (C₁-C₄)-alkoxy, CN, NO₂ or NH_{2;}
where the carbon chains or rings are unsubstituted or substituted by 1 - 11 fluorine atoms;
and its pharmaceutically acceptable salts, and the trifluoroacetic acid salts.

3. A compound of the formula I as claimed in claim 1 or 2 selected from the group consisting of:
trans-(2-chloro-6-trifluoromethylphenyl)-(octahydrobenzimidazol-2-ylidene)amine hydrochloride,
(S,S)-(2,6-dichlorophenyl)-(octahydrobenzimidazol-2-ylidene)amine trifluoroacetic acid salt,
cis-(2,6-dichlorophenyl)-(octahydrobenzimidazol-2-ylidene)amine trifluoroacetic acid salt,
(R,R)-(2,6-dichlorophenyl)-(octahydrobenzimidazol-2-ylidene)amine trifluoroacetic acid salt,
trans-(octahydrobenzimidazol-2-ylidene)-(2-phenoxyphenyl)amine hydrochloride,
trans-(2,6-dichlorophenyl)-(4,5-diisopropylimidazolidin-2-ylidene)amine hydrochloride,
trans-(2,6-dichlorophenyl)-(4,5-dicyclopropylimidazolidin-2-ylidene)amine trifluoracetic acid salt,
cis-(2,6-dichlorophenyl)-(4,5-dicyclopropylimidazolidin-2-ylidene)amine hydrochloride,
trans-(2,6-dichlorophenyl)-(4,5-diethylimidazolidin-2-ylidene)amine hydrochloride,
(2,6-dichlorophenyl)-(4,5-dimethylimidazolidin-2-ylidene)amine nitric acid salt,
trans-(2,6-dichlorophenyl)-(hexahydrocyclopentaimidazol-2-ylidene)amine trifluoroacetic acid salt.

4. A compound of the formula I as claimed in one or more of claims 1 to 3, selected from the group consisting of:
(S,S)-(2,6-dichlorophenyl)-(octahydrobenzimidazol-2-ylidene)amine trifluoroacetic acid salt,
cis-(2,6-dichlorophenyl)-(octahydrobenzimidazol-2-ylidene)amine trifluoroacetic acid salt,
(R,R)-(2,6-dichlorophenyl)-(octahydrobenzimidazol-2-ylidene)amine trifluoroacetic acid salt,
trans-(2,6-dichlorophenyl)-(4,5-diisopropylimidazolidin-2-ylidene)amine hydrochloride,
trans-(2,6-dichlorophenyl)-(4,5-dicyclopropylimidazolidin-2-ylidene)amine trifluoroacetic acid salt,
cis-(2,6-dichlorophenyl)-(4,5-dicyclopropylimidazolidin-2-ylidene)amine hydrochloride,
trans-(2,6-dichlorophenyl)-(4,5-diethylimidazolidin-2-ylidene)amine hydrochloride,
(2,6-dichlorophenyl)-(4,5-dimethylimidazolidin-2-ylidene)amine nitric acid salt,
trans-(2,6-dichlorophenyl)-(hexahydrocyclopentaimidazol-2-ylidene)amine trifluoroacetic acid salt.

5. The use of a compound of the formula I as claimed in claim 1 for preparing a medicament for the treatment or prophylaxis of disorders of the respiratory drive.

6. The use of a compound of the formula I as claimed in claim 1 for preparing a medicament for the treatment or prophylaxis of respiratory disorders, in particular respiratory disorders associated with sleeping, such as sleep apnea.

7. The use of a compound of the formula I as claimed in claim 1 for preparing a medicament for the treatment or prophylaxis of snoring.

8. The use of a compound of the formula I as claimed in claim 1 for preparing a medicament for the treatment or prophylaxis of acute and chronic renal diseases, in particular acute kidney failure and chronic kidney failure.

9. The use of a compound of the formula I as claimed in claim 1 for preparing a medicament for the treatment or prophylaxis of disorders of intestinal function.

10. The use of a compound of the formula I as claimed in claim 1 for preparing a medicament for the treatment or prophylaxis of disorders of gall function.

11. The use of a compound of the formula I as claimed in claim 1 for preparing a medicament for the treatment or prophylaxis of ischemic states of the peripheral and central nervous system and of stroke.

12. The use of a compound of the formula I as claimed in claim 1 for preparing a medicament for the treatment or prophylaxis of ischemic states of peripheral organs and limbs.

13. The use of a compound of the formula I as claimed in claim 1 for preparing a medicament for treating states of shock.

14. The use of a compound of the formula I as claimed in claim 1 for preparing a medicament for use in surgical operations and organ transplantations.

15. The use of a compound of the formula I as claimed in claim 1 for preparing a medicament for preserving and storing transplants for surgical interventions.

16. The use of a compound of the formula I as claimed in claim 1 for preparing a medicament for the treatment of diseases in which cell proliferation is a primary or secondary cause.

17. The use of a compound of the formula I as claimed in claim 1 for preparing a medicament for the treatment or prophylaxis of disorders of lipid metabolism.

18. The use of a compound of the formula I as claimed in claim 1 for preparing a medicament for the treatment or prophylaxis of infection by ectoparasites.

19. A medicament, comprising an effective amount of a compound of the formula I as claimed in one or more of claims 1 to 3.

## Revendications

1. Imidazolidines de formule 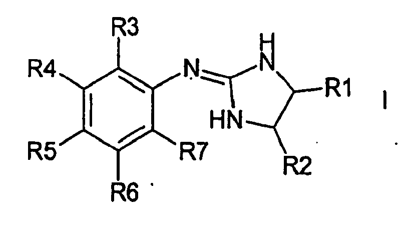 dans laquelle:
R1 et R2 signifient indépendamment l'un de l'autre CN, alkyle en C₁ à C₅, alcényle en C₂ à C₅, alcynyle en C₂ à C₅, cycloalkyle en C₃ à C₆ ou cycloalcényle en C₄ à C₆,
toutes les chaînes carbonées et tous les cycles carbonés étant non substitués ou substitués indépendamment l'un de l'autre par 1 à 11 atomes de fluor ou par jusqu'à deux radicaux choisis dans le groupe constitué par OH, NH₂, NHCH₃, N(CH₃)₂ et OCH₃;
ou
R1 et R2 signifient avec les deux atomes de carbone auxquels ils sont liés un cycle carboné de cinq à huit chaînons, saturé ou insaturé,
toutefois sans double liaison entre les deux atomes de carbone auxquels sont liés R1 et R2
et le cycle étant non substitué ou substitué par 1 à 12 atomes de fluor ou par jusqu'à deux radicaux choisis dans le groupe constitué par CH₃ et OCH₃;
R3 signifie F, Cl, Br, I, alkyle en C₁ à C₄, alcényle en C₁ à C₄, cycloalkyle en C₃ à C₆, OH, alcoxy en C₁ à C₄, O-phényle, CN, NO₂ ou NH₂;
où phényle est non substitué ou substitué par jusqu'à deux radicaux choisis dans le groupe constitué par CH₃, F, CI, Br, I, OH et OCH₃;
et
où les chaînes carbonées ou les cycles carbonés sont non substitués ou substitués par 1 à 11 atomes de fluor;
R4 à R6 signifient indépendamment l'un de l'autre H, F, Cl, Br, I, alkyle en C₁ à C₄, alcényle en C₁ à C₄, cycloalkyle en C₃ à C₆, OH, alcoxy en C₁ à C₄, CN, NO₂, NH₂, (alkyle en C₁ à C₄)amino ou (dialkyle en C₁ à C₄)amino;
où les chaînes carbonées ou les cycles carbonés sont non substitués ou substitués par 1 à 11 atomes de fluor;
R7 signifie H, F, Cl, Br, I, alkyle en C₁ à C₄, alcényle en C₁ à C₄, cycloalkyle en C₃ à C₆, OH, alcoxy en C₁ à C₄, CN, NO₂ ou NH₂,
où les chaînes carbonées ou les cycles carbonés sont non substitués ou substitués par 1 à 11 atomes de fluor;
ainsi que leurs sels pharmaceutiquement acceptables, ainsi que les sels de l'acide trifluoroacétique.

2. Composés de formule I selon la revendication 1, dans laquelle :
R1 et R2 signifient indépendamment l'un de l'autre alkyle en C₁ à C₅, alcényle en C₂ à C₅, alcynyle en C₂ à C₅, cycloalkyle en C₃ à C₆ ou cycloalcényle en C₄ à C₆, où toutes les chaînes carbonées et tous les cycles carbonés sont non substitués ou substitués indépendamment l'un de l'autre par 1 - 11 atomes de F ou par jusqu'à deux radicaux choisis dans le groupe constitué par NHCH₃, N(CH₃)₂ et OCH₃;
ou
R1 et R2 signifient avec les deux atomes de carbone auxquels ils sont liés un cycle carboné de cinq à huit chaînons, saturé ou insaturé,
toutefois sans double liaison entre les deux atomes de carbone auxquels sont liés R1 et R2
et
le cycle étant non substitué ou substitué par 1 à 12 atomes de fluor ou par jusqu'à deux radicaux choisis dans le groupe constitué par CH₃ et OCH₃;
R3 signifie F, Cl, Br, I, alkyle en C₁ à C₄, alcényle en C₁ à C₄, cycloalkyle en C₃ à C₆, OH, alcoxy en C₁ à C₄, O-phényle, CN, NO₂ ou NH₂;
où phényle est non substitué ou substitué par jusqu'à deux radicaux choisis dans le groupe constitué par CH₃, F, Cl, Br, OH et OCH₃;
et
où les chaînes carbonées ou les cycles carbonés sont non substitués ou substitués par 1 -11 atomes de F;
R4 à R6 signifient indépendamment l'un de l'autre H, F, Cl, Br, CH₃, OH, OCH₃, CN, NO₂, NH₂, NHCH₃, N(CH₃)₂;
où les groupes méthyle sont non substitués ou substitués par 1 - 3 atomes de F;
R7 signifie H, F, Cl, Br, I, alkyle en C₁ à C₄, alcényle en C₁ à C₄, cycloalkyle en C₃ à C₆, OH, alcoxy en C₁ à C₄, CN, NO₂ ou NH₂;
où les chaînes carbonées ou les cycles carbonés sont non substitués ou substitués par 1 à 11 atomes de fluor;
ainsi que leurs sels pharmaceutiquement acceptables, ainsi que les sels de l'acide trifluoroacétique.

3. Composés de formule I selon l'une ou plusieurs des revendications 1 et 2, qui sont:
le chlorhydrate de la trans-(2-chloro-6-trifluorométhyl-phényl)-(octahydrobenzoimidazol-2-ylidén)-amine,
le sel de l'acide trifluoroacétique de la (S,S)-(2,6-dichlorophényl)-(octahydrobenzoimidazol-2-ylidén)-amine,
le sel de l'acide trifluoroacétique de la cis-(2,6-dichlorophényl)-(octahydrobenzoimidazol-2-ylidén)-amine,
le sel de l'acide trifluoroacétique de la (R,R)-(2,6-dichlorophényl)-(octahydrobenzoimidazol-2-ylidén)-amine,
le chlorhydrate de la trans-(octahydro-benzoimidazol-2-ylidène)-(2-phénoxy-phényl)-amine,
le chlorhydrate de la trans-(2,6-dichlorophényl)-(4,5-diisopropyl-imidazolidin-2-ylidén)-amine,
le sel de l'acide trifluoroacétique de la trans-(2,6-dichlorophényl)-(4,5-dicyclopropylimidazolidin-2-ylidén)-amine,
le chlorhydrate de la cis-(2,6-dichlorophényl)-(4,5-dicyclopropyl-imidazolidin-2-ylidén)-amine,
le chlorhydrate de la trans-(2,6-dichlorophényl)-(4,5-diéthyl-imidazolidin-2-ylidén)-amine,
le sel de l'acide nitrique de la (2,6-dichlorophényl)-(4,5-diméthyl-imidazolidin-2-ylidén)-amine,
le sel de l'acide trifluoroacétique de la trans-(2,6-dichlorophényl)-(hexahydrocyclopentaimidazol-2-ylidén)-amine.

4. Composés de formule I selon l'une ou plusieurs des revendications 1 à 3, qui sont:
le sel de l'acide trifluoroacétique de la (S,S)-(2,6-dichlorophényl)-(octahydrobenzoimidazol-2-ylidén)-amine,
le sel de l'acide trifluoroacétique de la cis-(2,6-dichlorophényl)-(octahydrobenzoimidazol-2-ylidén)-amine,
le sel de l'acide trifluoroacétique de la (R,R)-(2,6-dichlorophényl)-(octahydrobenzoimidazol-2-ylidén)-amine,
le chlorhydrate de la trans-(2,6-dichlorophényl)-(4,5-diisopropyl-imidazolidin-2-ylidén)-amine,
le sel de l'acide trifluoroacétique de la trans-(2,6-dichlorophényl)-(4,5-dicyclopropylimidazolidin-2-ylidén)-amine,
le chlorhydrate de la cis-(2,6-dichlorophényl)-(4,5-dicyclopropyl-imidazolidin-2-ylidén)-amine,
le chlorhydrate de la trans-(2,6-dichlorophényl)-(4,5-diéthyl-imidazolidin-2-ylidén)-amine,
le sel de l'acide nitrique de la (2,6-dichlorophényl)-(4,5-diméthyl-imidazolidin-2-ylidén)-amine,
le sel de l'acide trifluoroacétique de la trans-(2,6-dichlorophényl)-(hexahydrocyclopentaimidazol-2-ylidén)-amine.

5. Utilisation d'un composé de formule I selon la revendication 1 pour préparer un médicament destiné au traitement ou à la prophylaxie de troubles de la fonction respiratoire.

6. Utilisation d'un composé de formule I selon la revendication 1 pour préparer un médicament destiné au traitement ou à la prophylaxie de troubles de la respiration, en particulier les troubles de la respiration liés au sommeil, tels que les apnées du sommeil.

7. Utilisation d'un composé de formule I selon la revendication 1 pour préparer un médicament destiné au traitement ou à la prophylaxie du ronflement.

8. Utilisation d'un composé de formule I selon la revendication 1 pour préparer un médicament destiné au traitement ou à la prophylaxie de maladies rénales aiguës et chroniques, en particulier de l'insuffisance rénale aiguë et de l'insuffisance rénale chronique.

9. Utilisation d'un composé de formule I selon la revendication 1 pour préparer un médicament destiné au traitement ou à la prophylaxie de troubles de la fonction intestinale.

10. Utilisation d'un composé de formule I selon la revendication 1 pour préparer un médicament destiné au traitement ou à la prophylaxie de troubles de la fonction de la vésicule biliaire.

11. Utilisation d'un composé de formule 1 selon la revendication 1 pour préparer un médicament destiné au traitement ou à la prophylaxie d'états ischémiques du système nerveux central et périphérique et de l'attaque.

12. Utilisation d'un composé de formule I selon la revendication 1 pour préparer un médicament destiné au traitement ou à la prophylaxie d'états ischémiques d'organes périphériques et des membres.

13. Utilisation d'un composé de formule I selon la revendication 1 pour préparer un médicament destiné au traitement d'états de choc.

14. Utilisation d'un composé de formule I selon la revendication 1 pour la préparation d'un médicament destiné à être utilisé lors d'opérations chirurgicales et de transplantations d'organes.

15. Utilisation d'un composé de formule I selon la revendication 1 pour la préparation d'un médicament destiné à la conservation et au stockage de transplants pour des interventions chirurgicales.

16. Utilisation d'un composé de formule I selon la revendication 1 pour la préparation d'un médicament destiné au traitement de maladies dans lesquelles la prolifération cellulaire est une cause primaire ou secondaire.

17. Utilisation d'un composé de formule I selon la revendication 1 pour la préparation d'un médicament destiné au traitement ou la prophylaxie de troubles du métabolisme lipidique.

18. Utilisation d'un composé de formule I selon la revendication 1 pour la préparation d'un médicament destiné au traitement ou à la prophylaxie de l'attaque par des ectoparasites.

19. Remède contenant une quantité active d'un composé de formule I selon l'une ou plusieurs des revendications 1 à 3.
